# EUROPEAN PATENT APPLICATION

(11) **EP 4 306 549 A1**
(43) Date of publication of application: **17.01.2024**
(21) Application number: 22184568.8
(22) Date of filing: 13.07.2022
(51) Int. Cl.: C08B 37/02, C08J 3/075, C08L 5/02, A61K 47/36, A61L 27/52

(54) **HYDROGELS FOR CELL THERAPY**

(71) Applicant: Adocia, 69003 Lyon (FR)
(72) Inventor: GEISSLER, Alexandre, 69330 Meyzieu (FR); LAURENT, Nicolas, 01700 Miribel (FR); PLANCQ, Baptiste, 01700 Saint Maurice de Beynost (FR); SOULA, Gérard, 69330 Meyzieu (FR); ELOY, Marie-Rose, 69006 Lyon (FR)
(74) Representative: Tripoz, Inès

(57) **Abstract**

The invention concerns cross-linked dextran polymer Dx- bearing anionic groups wherein the at least divalent radical L(-)i is covalently bound to the dextran polymer backbone with i -W- radicals, wherein,
- L(-)i is a linear or branched poly(oxazoline)
- i is the valence of L and the number of -W- radicals bound to the dextran polymer and is an integer comprised from 2 to 8 (2 ≤ i ≤ 8),
- -(A-f₂)a-G₁- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

It also concerns the dextran polymers before the crosslinking reaction and the process to synthetize a cross-linked dextran polymer according to the invention, into the form of a hydrogel.

It also concerns a hydrogel comprising the crosslinked dextran polymer of the invention and/or a hydrogel that further comprises biological cells.

It also concerns therapeutic use of the hydrogel according to the invention for treating a disorder or disease in a mammal wherein the disorder or disease is due to lack or malfunction of endocrine function of pancreas organ and an implant comprising the hydrogel according to the invention.

## Description

The domain of the invention is therapy, in particular cell therapy. More particularly the invention is about an implant comprising a hydrogel which may incorporate:
- active principles, such as peptides, hormones or proteins, or
- secreting cells, which may be cells secreting peptides or hormones.

The aim is to prevent, treat or cure disease. In particular, this could allow the prevention and/or treatment of chronic diseases by replacing totally or in part the function of naturally occurring cells which are deficient in the patients. The invention is also about a crosslinked polymer, its precursors, a process for obtaining the crosslinked polymer and a process for obtaining a hydrogel, in particular a hydrogel containing cells.

The cells may be isolated or aggregated and may be of one type or of different types.

Hydrogels can be used in multiple systems like:
- scaffolds as controlled drug or active pharmaceutical ingredient release systems or
- scaffolds to be used as implantable device comprising cells.

Hydrogels comprise or consist of polymers that are crosslinked in a 3D network. They can either be natural or synthetic, homopolymers or copolymers. They have the ability to absorb and retain large amounts of water. This is known as the swelling of hydrogels.

In order to have a system which may be an implant able to deliver active principle on the long run, many features have to be obtained.

Among these features may be cited:
- a low degradability, in particular a low biodegradability, or no biodegradability, or a good in vivo stability, in order for the incorporated cells not to escape in the organism of the patient and the host cells not to penetrate in the implant,
- a good permselectivity, defined as the selective permeability toward biological elements according to their size or molecular weight, allowing a low, or even better no, immune response by isolating the incorporated cells, totally or in part, from the immune system of the host while allowing the passage of the active principle, for example a hormone, peptide or protein,
- a good mitigation of the foreign body response, or a good biocompatibility, in particular a low cytotoxicity and a good local tolerance,
- allowing the cells to have a high survival rate, such as having a good vascularisation close to the cells and sufficient flux of nutrients to the cells,
- allowing the cells to have a good functionality within the hydrogel.

In order to be used as controlled release systems or scaffolds for cells, hydrogels must have particular characteristics so as to exhibit all or part of the desired properties such as disclosed above as well as good mechanical and rheological properties.

Among the rheological and mechanical properties that are of high interest for the hydrogel may be cited:
- a good homogeneity, which can be linked to a good transparency or translucency,
- appropriate resistance and flexibility toward stress and strain mechanics, in particular when being handled and implanted, , for example through laparoscopy,
- a defined mesh size to maximise oxygen and nutrients exchanges, controlled transport properties and permselectivity
- a good stability in vivo, i.e. resistance to hydrolytic, enzymatic or oxidative degradation.

Among parameters which can give indications on the rheological and mechanical desired properties may be cited:
- tan δ (called loss tangent) which gives indication on mechanical properties,
- G', which gives indication on the elastic modulus (stiffness), and on the mesh size,
- compression and/or traction deformation at break, which gives indication on the elasticity and resistance of the hydrogel,
- swellability, which gives indications on the water content, dimensions and mechanical properties.

Among the problems to be solved is obtaining a hydrogel with properties allowing:
- a manipulation for implanting the hydrogel without breaking it, such as by laparoscopy, and/or
- a gel to remain in place after implantation, for example allowing the hydrogel not to get folded after implantation and/or to be immobilized relative to the tissue on which it is implanted.

Another problem to be dealt with relates to the sedimentation of the cells, or islets during the crosslinking leading to gelation.

In the prior art hydrogels comprising cells which are crosslinked are very often intended for allowing the growth of a cell object, for example 3D cell culture. This kind of application needs that the hydrogels could at the same time embed the starting cells and make space for the new cells obtained by outgrowth and/or proliferation. In order to comply with these two opposite characteristics, the solution is to have degradable, for example via cleavable bonds, hydrogels which are strong enough to embed the cells and which upon degradation make sufficient space for new cells. A method of choice to obtain this degradation is to have a peptidic structure within the crosslinked hydrogel, in particular at the level of the crosslinker in between the polymeric backbone.

This type of behavior is completely incompatible with the aim of the instant invention which is to obtain a long lasting crosslinked hydrogel encapsulating/embedding cells. In this case the hydrogel needs to have very little, or even better non-degradable, as this key feature will allow to keep the cells invisible from the immune.

The underlying problem is solved by the provision of a gel that presents physicochemical properties to allow the manufacture of an implantable device and biocompatibility properties that allow the cells survival.

Moreover, and on the contrary to many prior arts, the invention allows the preparation of hydrogels which have tunable features, considering the precursors used and the way the crosslinking is performed. This can lead to hydrogels having a controlled incorporation and release of specific objects from the hydrogel.

The suitability of the hydrogel depends on its bulk structure, thus important parameters used to characterize the network structure of the hydrogel according to the invention are the polymer volume fraction in the swollen state, the molecular weight of the polymer chain between two neighboring cross-linking points, and the corresponding mesh size.

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a poly(oxazoline) (POx)chain.

The properties of this family of hydrogels are tunable and tailorable to the applications by choosing and adapting the cross-linking reaction conditions, the substitution degree and molecular weight of the dextrans and cross-linkers.

The cross-linked dextran polymer according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment -W- comprises at most 60 carbon atoms.

In an embodiment -W- comprises at most 60 carbon atoms without counting any -CH₂-CH₂O- radicals.

In an embodiment -W- comprises at most 50 carbon atoms.

In an embodiment -W- comprises at most 50 carbon atoms without counting any -CH₂-CH₂O- radicals.

In an embodiment -W- comprises at most 40 carbon atoms.

In an embodiment -W- comprises at most 40 carbon atoms without counting any -CH₂-CH₂O- radicals.

In an embodiment -W- comprises at most 30 carbon atoms.

In an embodiment -W- comprises at most 30 carbon atoms without counting any -CH₂-CH₂O- radicals.

In an embodiment -W- comprises at most 20 carbon atoms.

In an embodiment -W- comprises at most 20 carbon atoms without counting any -CH₂-CH₂O- radicals.

In an embodiment -W- comprises at most 10 carbon atoms.

In an embodiment -W- comprises at most 10 carbon atoms without counting any -CH₂-CH₂O- radicals.

In an embodiment W comprises at most 5 oxygen atoms.

In an embodiment W comprises at most 5 oxygen atoms without counting any -CH₂-CH₂O- radicals.

The cross-linked dextran polymer according to the invention is a dextran polymer Dx- bearing anionic groups wherein an at least divalent radical L(-)ᵢ is covalently bound to the dextran polymer backbone with i radicals, chosen among the dextrans of formula I,

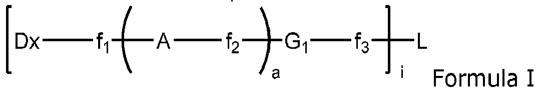

Wherein :
- a is an integer equal to 0 or 1.
- i is an integer comprised from 2 to 8, (2 ≤ i ≤ 8).
- L can be linked to the same [Dx-f₁-(A-f₂)ₐ-G₁-f₃] radicals, or to different ones.
- Dx- is a dextran moiety, which can be substituted by specific anionic groups in salified form, and optionally by alkyl carboxylate derivatives in salified form.
- f₁ is an ether function.
- The divalent radical -A- is a linear, -(CH₂)ₙ₁- with n₁ an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5)
- f₂ is an amide function.
- The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms. In a preferred embodiment, -G₁- is a succinimide derivative, or an alkyl sulfone derivative which can contain one heteroatom such as oxygen or sulphur, or a 1,4-triazole derivative.
- The integer i is the valence of the central-linker L, and the number of, identical or different, [Dx-f₁-(A-f₂)ₐ-G₁-f₃] radicals connected to L.
- f₃ is an amine function, or a thioether function, or an ether function, or an amide function, or a carbamate function, or a carbon-nitrogen covalent bond, or carbon-aromatic carbon covalent bond.
- The central-linker L is a poly(oxazoline) (POx) derivative, which can be linear or branched.

In one embodiment, the crosslinked dextran hydrogel according to the invention is a dextran polymer wherein the central-linker L is a linear, or a branched POx radical.

In an embodiment, the cross-linked dextran polymer according to the invention is a dextran polymer wherein L is a linear or branched POx radical comprising at most 8 arms, which number-average molecular weight (Mn) is comprised from 500 to 40 000 g/mol (500 ≤ Mn ≤ 40 000 g/mol).

In an embodiment, the cross-linked dextran polymer according to the invention is a dextran polymer wherein L is a linear or branched POx radical comprising at most 8 arms, which number-average molecular weight (Mn) is comprised from 1000 to 25 000 g/mol (500 ≤ Mn ≤ 40 000 g/mol).

In one embodiment, the POx central linker is a 2-arm POx, chosen among the linkers of formula II.

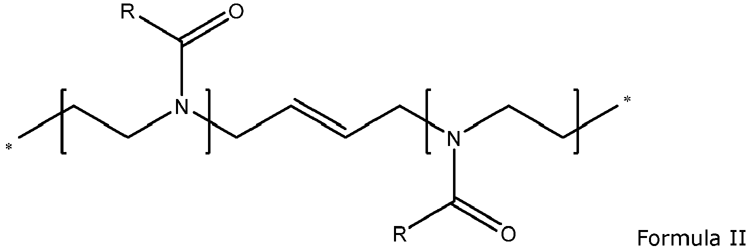

Wherein:
- The radical -R is a linear, -(CH₂)ₙ₁-CH₃ with n₁ an integer comprised from 0 to 4 (0 ≤ n₁ ≤ 4), branched, or cyclic alkyl derivative.
- The ^{∗} represents the sites of f₃, which is an amine function, or an ether function, or a thioether function, or an amide function, or a carbamate function, or a carbon-nitrogen covalent bond, or a carbon-aromatic carbon covalent bond.

In one embodiment, the POx central linker is a 4-arm POx, chosen among the linkers of formula III. 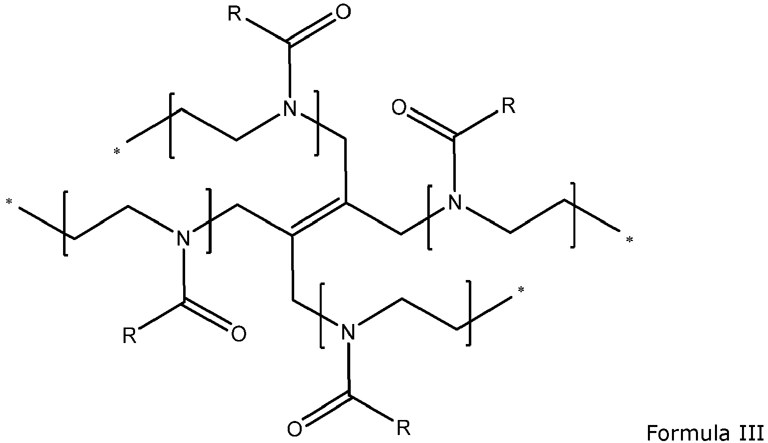 Wherein:
- The radical -R is a linear, -(CH₂)ₙ₁-CH₃ with n₁ an integer comprised from 0 to 4 (0 ≤ n₁ ≤ 4), branched, or cyclic alkyl derivative.
- The ^{∗} represents the sites of f₃, which is an amine function, or an ether function, or a thioether function, or an amide function, or a carbamate function, or a carbon-nitrogen covalent bond, or a carbon-aromatic carbon covalent bond.

In another embodiment, the POx central linker is a 4-arm POx, chosen among the linkers of formula IV:

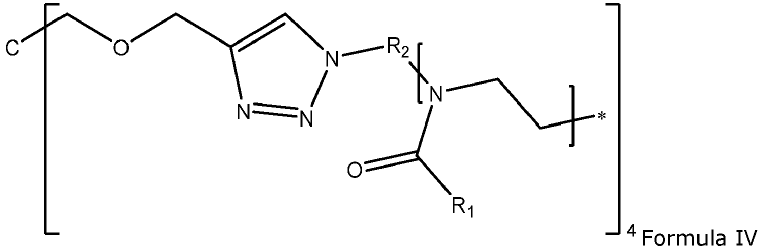

Wherein:
- The radical -R₁ is a linear, -(CH₂)ₙ₁-CH₃ with n₁ an integer comprised from 0 to 4 (0 ≤ n₁ ≤ 4), branched, or cyclic alkyl derivative.
- The divalent radical -R₂- is a linear, -(CH₂)ₙ₂- with n₂ an integer comprised from 2 to 6 (2 ≤ n₂ ≤ 6).
- The ^{∗} represents the sites of f₃, which is an amine function, or an ether function, or a thioether function, or an amide function, or a carbamate function, or a carbon-nitrogen covalent bond, or a carbon-aromatic carbon covalent bond.

In another embodiment, the POx central linker is a 4-arm POx, chosen among the linkers of formula V:

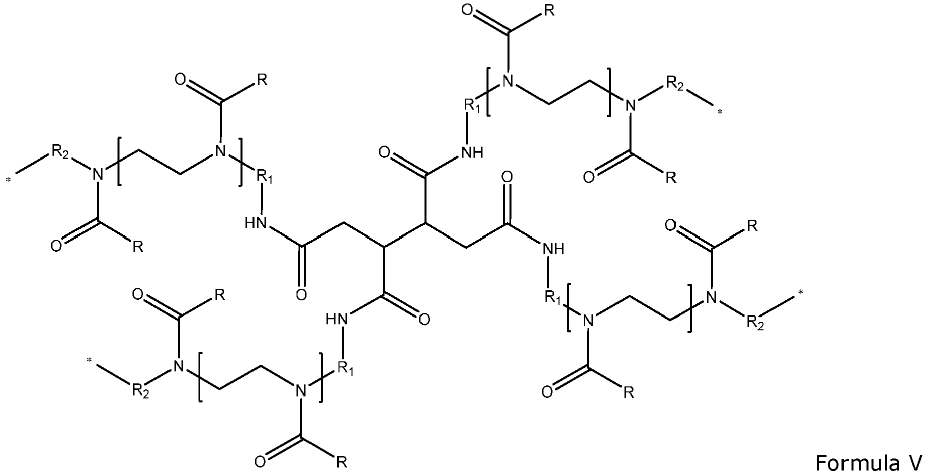

Wherein:
- The radical -R is a linear, -(CH₂)ₙ₁-CH₃ with n₁ an integer comprised from 0 to 4 (0 ≤ n₁ ≤ 4), branched, or cyclic alkyl derivative.
- In one embodiment, R₁ = -CH₂-CH₂- and R₂ is a linear, -(CH₂)ₙ₂- with n₂ an integer comprised from 2 to 6 (2 ≤ n₂ ≤ 6)
- In another embodiment, R₂ = -CH₂-CH₂- and R₁ is a linear, ^{∗}-(CH₂)ₙ₂-^{∗} with n₂ an integer comprised from 2 to 6 (2 ≤ n₂ ≤ 6)
- The ^{∗} represents the sites of f₃, which is an amine function, or an ether function, or a thioether function, or an amide function, or a carbamate function, or a carbon-nitrogen covalent bond, or a carbon-aromatic carbon covalent bond.

The hydroxyl functions of the dextran polymer Dx- can be functionalised by at least one specific anionic group such as: sulphate anions, or sulfonate anions, or phosphate anions, or phosphonate anions.

In one embodiment, the hydroxyl functions of the dextran polymer Dx- can be functionalised by sulphate anions in salified form, and optionally by alkyl carboxylate derivatives in salified form.

In another embodiment, the hydroxyl functions of the dextran polymer Dx- can be functionalised by sulfonate anions in salified form, and optionally by alkyl carboxylate derivatives in salified form.

In another embodiment, the hydroxyl functions of the dextran polymer Dx-, can be functionalised by phosphate anions in salified form, and optionally by alkyl carboxylate derivatives in salified form.

In another embodiment, the hydroxyl functions of the dextran polymer Dx- can be functionalised by phosphonate anions in salified form, and optionally by alkyl carboxylate derivatives in salified form.

In another embodiment, the hydroxyl functions of the dextran polymer Dx- can be functionalised by alkyl carboxylate derivatives in salified form.

The specific anionic groups defined previously are chosen among the groups of formula X:

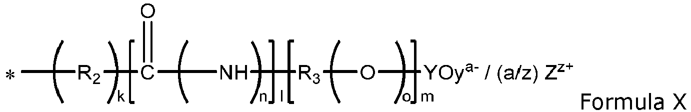

Wherein:
- ^{∗} represents the link to the O atoms of the dextran to form an ether function.
- y=2 or 3.
- When y=2, alkyl carboxylate derivatives, then:
   ∘ Y=C and a=1.
   ∘ k=1, l=0 and m=0.
   ∘ R2=Alkyl.
- When y=3, anionic group, then:
   ∘ Y=S and a=1, or Y=P and a=2.
   ∘ k=0 or 1.
   ∘ l=0 or 1.
   ∘ m=0 or 1.
   ∘ n=1 or 2, in particular 1.
   ∘ o=0 or 1.
   ∘ if l=1 then m=1.
   ∘ R₃=linear, branched, or cyclic alkyl which may contain one heteroatom such as nitrogen, or aromatic, or PEG.
   ∘ R₂=Alkyl.
- And, Z is a counter ion, which can be an alkali metal and z=1, or which can be an alkaline earth metal and z=2.

In a preferred embodiment, the dextran backbone, Dx-, can be functionalised by sulphate anions in salified form, and optionally by alkyl carboxylate derivatives in salified form.

In another preferred embodiment, the dextran backbone, Dx-, can be functionalised by alkyl sulfonate anions in salified form, and optionally by alkyl carboxylate derivatives in salified form.

In another preferred embodiment, the dextran backbone, Dx-, can be functionalised by sulfonate anions in salified form, supported by an alkyl chain comprising a dimethyl-ammonium cation, and optionally by alkyl carboxylate derivatives in salified form.

In another preferred embodiment, the dextran backbone, Dx-, can be functionalised by alkyl carboxylate derivatives in salified form.

In an embodiment, the cross-linked dextran polymer bearing anionic groups according to the invention is a dextran polymer wherein the dextran polymer backbone is according to formula XI, 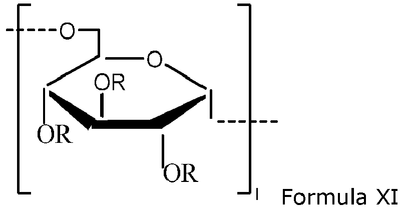 wherein R is chosen among
- -H, a anionic group of formula X, or a -(A-f₂)ₐ-G₁- radical bearing a L(-)ᵢ crosslinker,
- I is comprised from 20 to 5000 (20 ≤ I ≤ 5000),
- -(A-f₂)ₐ-G₁- and L(-)_{I} radicals having the previously defined meanings.

In an embodiment, the cross-linked dextran polymer according to the invention is a dextran polymer wherein the dextran polymer backbone is a dextran having a weight average molecular weight (Mw) comprised from 5 to 1000 kDa before cross-linking and substitution.

In other words, the cross-linked dextran polymer according to the invention is obtained after substitution and crosslinking of a native dextran polymer having a weight average molecular weight (Mw) comprised from 5 to 1000 kDa.

In an embodiment, the cross-linked dextran polymer according to the invention is a dextran polymer wherein the dextran polymer backbone is a dextran having a weight average molecular weight (Mw) comprised from 5 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 25 kDa before cross-linking and substitution.

In an embodiment, the cross-linked dextran polymer according to the invention is a dextran polymer wherein the dextran polymer backbone is a dextran having a weight average molecular weight (Mw) comprised from 250 to 1000 kDa before cross-linking and substitution.

In an embodiment, the cross-linked dextran polymer according to the invention is a dextran polymer wherein the dextran polymer backbone is a dextran having a weight average molecular weight (Mw) comprised from 10 to 500 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 500 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 100 kDa before cross-linking and substitution.

In an embodiment, the cross-linked dextran polymer according to the invention is a cross-linked dextran polymer wherein the degree of substitution (DS₁) of the dextran backbone with the a -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution (DS₁) of the dextran backbone with the -(A-f₂)ₐ-G₁- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the degree of substitution (DS₁) of the dextran backbone with the -(A-f₂)ₐ-G₁- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the degree of substitution (DS₁) of the dextran backbone with the -(A-f₂)ₐ-G₁- radical bearing a L(-)ᵢ crosslinker is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 5 to 250 kDa before cross-linking and substitution and the degree of substitution (DS₁) of the dextran backbone with the -(A-f₂)ₐ-G₁- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 20 to 100 kDa before cross-linking and substitution and the degree of substitution (DS₁) of the dextran backbone with the -(A-f₂)ₐ-G₁- radical bearing a L(-)ᵢ crosslinker groups is comprised in the range from 0.2 to 0.4 (0.2 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 20 to 100 kDa before cross-linking and substitution and the degree of substitution (DS₁) of the dextran backbone with the --(A-f₂)ₐ-G₁- radical bearing a L(-)ᵢ crosslinker is comprised in the range from 0.2 to 0.3 (0.2 ≤ DS₁ ≤ 0.3).

In an embodiment, the cross-linked dextran polymer according to the invention is a cross-linked dextran polymer wherein the dextran polymer backbone is a dextran having a weight average molecular weight (Mw) is from 250 to 1000 kDa before cross-linking and substitution and the degree of substitution (DS₁) of the dextran backbone with the -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution (DS₁) of the dextran backbone with the -(A-f₂)ₐ-G₁- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution (DS₁) of the dextran backbone with the -(A-f₂)ₐ-G₁- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution (DS₁) of the dextran backbone with the -(A-f₂)ₐ-G₁- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the degree of substitution (DS₄) of the dextran backbone with a radical of formula X is comprised in the range from 0.5 to 3 (0.5 ≤ DS₄ ≤ 3).

In an embodiment, the cross-linked dextran polymer according to the invention is a cross-linked dextran polymer wherein the degree of substitution (DS₄) of the dextran backbone with a radical of formula X is comprised in the range from 1 to 2.75 (1 ≤ DS₄ ≤ 2.75).

In an embodiment, the cross-linked dextran polymer according to the invention is a cross-linked dextran polymer wherein the degree of substitution (DS₄) of the dextran backbone with a radical of formula X is comprised in the range from 1.5 to 2.5 (1.5 ≤ DS₄ ≤ 2.5).

In an embodiment, the cross-linked dextran polymer according to the invention is a cross-linked dextran polymer wherein the degree of substitution (DS₄) of the dextran backbone with a radical of formula X is comprised in the range from 1.75 to 2.25 (1.75 ≤ DS₄ ≤ 2.25).

In an embodiment, the cross-linked dextran polymer according to the invention is a cross-linked dextran polymer wherein the degree of substitution of carboxylate (DS_{c}) of the dextran backbone is comprised in the range from 0.2 to 3 (0.2 ≤ DS_{c} ≤ 3).

In an embodiment, the cross-linked dextran polymer according to the invention is a cross-linked dextran polymer wherein the degree of substitution of carboxylate (DS_{c}) of the dextran backbone is comprised in the range from 0.3 to 2.5 (0.3 ≤ DS_{c} ≤ 2.5).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.2 to 2.5 (0.2 ≤ DS₃ ≤ 2.5).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.3 to 2.0 (0.3 ≤ DS₃ ≤ 2.0).

In an embodiment, the cross-linked dextran polymer according to the invention is a cross-linked dextran polymer having a molar ratio (DC) between the molar concentration of the -(A-f₂)ₐ-G₁- radical and the molar concentration of the reactive functions of the cross-linker L(-)ᵢ comprised in a range from 0.5 to 1.5 (0.5 ≤ DC ≤ 1.5).

In an embodiment, the cross-linked dextran polymer according to the invention is a cross-linked dextran polymer having a molar ratio between the molar concentration of the -(A-f₂)ₐ-G₁- radical and the molar concentration of the reactive functions of the cross-linker L(-)ᵢ is comprised in a range from 0.8 to 1.2 (0.8 ≤ DC ≤ 1.2).

In an embodiment, the cross-linked dextran polymer according to the invention is a cross-linked dextran polymer having a molar ratio between the molar concentration of the -(A-f₂)ₐ-G₁- radical and the molar concentration of the reactive functions of the cross-linker L(-)ᵢ is comprised in a range from 0.9 to 1.1 (0.9 ≤ DC ≤ 1.1).

In an embodiment, the cross-linked dextran polymer according to the invention is a cross-linked dextran polymer having a molar ratio between the molar concentration of the -(A-f₂)ₐ-G₁- radical and the molar concentration of the reactive functions of the cross-linker L(-)ᵢ is comprised in a range from 0.95 to 1.05 (0.95 ≤ DC ≤ 1.05).

In an embodiment, the cross-linked dextran polymer according to the invention is a cross-linked dextran polymer having a molar ratio between the molar concentration of the -(A-f₂)ₐ-G₁- radical and the molar concentration of the reactive functions of the cross-linker L(-)ᵢ is 1 (DC = 1).

In an embodiment, the cross-linked dextran polymer according to the invention is a cross-linked dextran polymer obtained from a reaction between the reactive function of the -W- precursor and the reactive function of the (L)(-)i precursor where reactive functions are present in the same concentration (DC = 1) and are comprised in a range going from 5 to 25 mM.

In an embodiment they are comprised in the range of 5 to 10 mM.

In an embodiment they are comprised in the range of 10 to 15 mM.

In an embodiment they are comprised in the range of 15 to 20 mM.

In an embodiment they are comprised in the range of 20 to 25 mM.

In one embodiment -A-, is a linear, -(CH₂)ₙ₁- with n₁ an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5).

The nature of radical G1 depends of the crosslinking process, below are described the different crosslinking process together with the G1 radicals.

In one embodiment, the integer a=1, the crosslinking process is realised with a Michael addition with maleimide derivatives, or vinyl sulfone derivatives.

In one embodiment, the divalent radical -G₁- is a succinimide derivative according to the following formula:

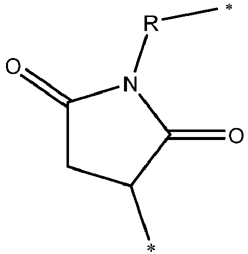

Wherein:
- R is a linear, branched, or cyclic alkyl derivative, or R is an aromatic, or R is a PEG derivative.
- The ^{∗} represent the sites of f₂, which is an amide function, and f₃, which is an amine function, or an ether function, or a thioether function.

In another embodiment, the divalent radical -G₁- is a succinimide derivative according to the following formula:

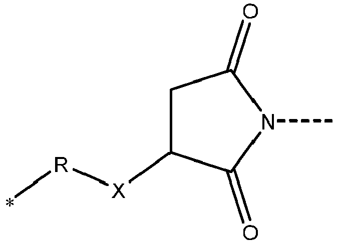

Wherein:
- X is an oxygen atom, or a sulphur atom, or a nitrogen atom.
- R is a linear, branched, or cyclic alkyl derivative, or R is a PEG derivative.
- The ^{∗} represents the site of f₂, which is an amide function, and the dotted bond represents f₃, which is a carbon-nitrogen covalent bond.

In another embodiment, the divalent radical -G₁- is a sulfone derivative according to the following formula:

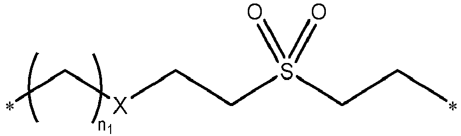

Wherein:
- n₁ is an integer comprised from 0 to 7 (0 ≤ n₁ ≤ 7).
- X is an oxygen atom, or a sulphur atom, or a CH₂ group.
- The ^{∗} represent the sites of f₂, which is an amide function, and f₃, which is an amine function, or an ether function or a thioether function.

In one embodiment, the crosslinking process is realised with a 1,3-cycloaddition between alkyne and azide derivatives, known as 1,3-dipolar cycloaddition or Huisgen reaction.

In one embodiment, the integer a=1, the divalent radical -G₁- is a 1,4-triazole derivative according to the following formula:

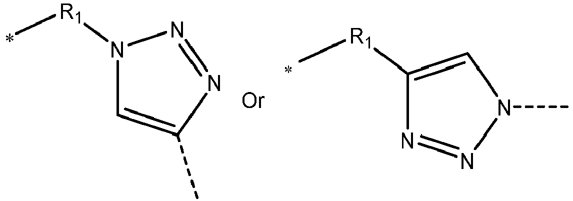

Wherein:
- R₁ is a linear, branched, or cyclic alkyl derivative, which can contain heteroatom such as oxygen, or R₁ is an aromatic derivative, or R₁ is a PEG derivative.
- The ^{∗} represents the site of f₂, which is an amide function, and the dotted bond represents f₃, which is either a carbon-nitrogen covalent bond or a carbon-aromatic carbon covalent bond.

In another embodiment, the integer a=0, the divalent radical -G₁- is a 1,4-triazole derivative according to the following formula:

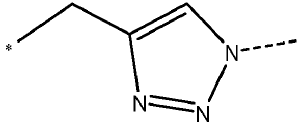

Wherein:
- The ^{∗} represents the site of f₁, which is an ether function, and the dotted bond represents f₃, which is a carbon-nitrogen covalent bond.

In one embodiment, the integer a=1, the crosslinking process is realised with a 1,3-cycloaddition between strain alkyne and azide derivatives, known as Strainpromoted azide-alkyne cycloaddition or SPAAC.

In another embodiment, the divalent radical -G₁- is a triazole derivative according to the following formula:

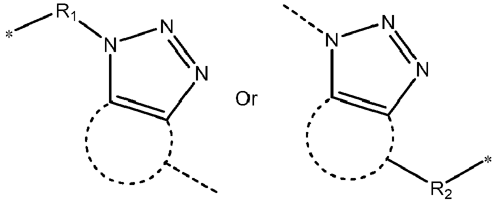

Wherein:
- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- R₁ is a linear, branched, or cyclic alkyl derivative, which can contain heteroatom such as oxygen, or R₁ is an aromatic derivative, or R₁ is a PEG derivative.
- R₂ is a linear, branched, or cyclic alkyl derivative, which can contain heteroatom such as oxygen, or R₂ is an aromatic derivative, or R₂ is a PEG derivative.
- The ^{∗} represents the site of f₂, which is an amide function, and the dotted bond represents f₃, which is a carbon-nitrogen covalent bond, or an amide function, or a carbamate function.

In a preferred embodiment, the divalent radical -G₁- is a triazole derivative according to the following formula:

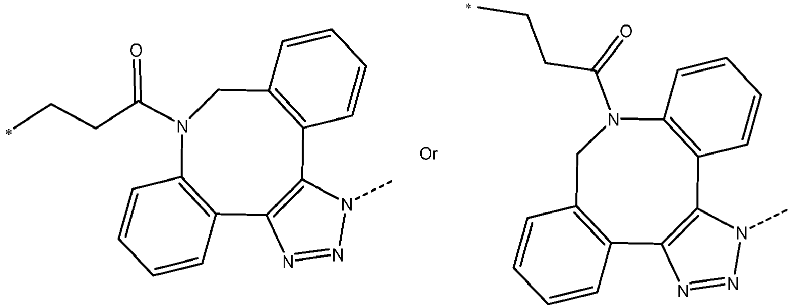

Wherein:
- The ^{∗} represents the site of f₂, which is an amide function, and the dotted bond represents f₃, which is a carbon-nitrogen covalent bond.

In another preferred embodiment, the divalent radical -G₁- is a triazole derivative according to the following formula:

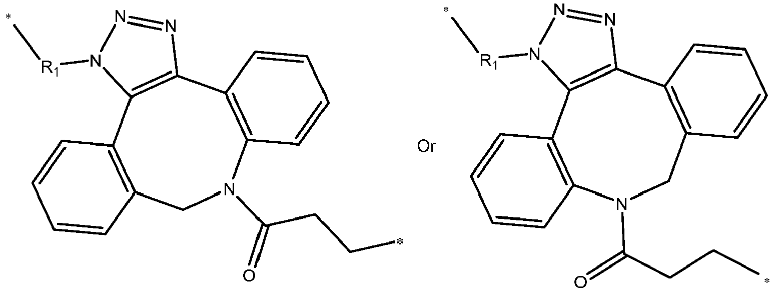

Wherein:
- R₁ is a linear, branched, or cyclic alkyl derivative, which can contain heteroatom such as oxygen, or R₁ is an aromatic derivative, or R₁ is a PEG derivative.
- The ^{∗} represent the sites of f₂ and f₃, which are amide functions.

In one embodiment, f₃ is an amine function.

In another embodiment, f₃ is an ether function.

In another embodiment, f₃ is a thioether function.

In another embodiment, f₃ is an amide function.

In another embodiment, f₃ is a carbamate function.

In another embodiment, f₃ is a carbon-nitrogen covalent bond.

In another embodiment, f₃ is a carbon-aromatic carbon covalent bond.

The invention also concerns the dextran polymers of formula VIII before the crosslinking reaction. 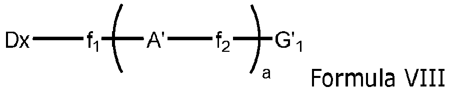 Wherein
- f₁, f₂, Dx are as defined above if a is not equal to 0,
- and
- if a is equal to 0, f₁ is an ether function and G'₁ is a propargylic derivative,
- if a is not equal to 0 -A' is A as defined above.

In an embodiment, if a=1, A' is an alkyl carboxylate derivative, or a 2-hydroxyalkyl carboxylate, or a 2-hydroxyalkylamine.

A' as carboxylate derivatives can be formalized with the following formula:

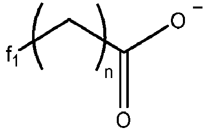

A' as a 2-hydroxyalkyl carboxylate derivative can be formalized with the following formula:

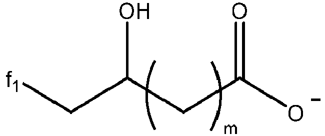

A' as a 2-hydroxyalkylamine derivative can be formalized with the following formula:

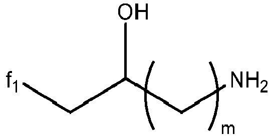

Wherein:
- n is an integer comprised from 1 to 7 (1 ≤ n ≤ 7)
- m is an integer comprised from 1 to 5 (1 ≤ m ≤ 5)
- f₁ is defined as previously

In an embodiment, if a=1, G'1 is a maleimide derivative, or a vinylsulfone derivative, or a strained cyclooctyne derivative, or an azide derivative, or propargyl derivative, or a thiol derivative, or an amine derivative, or a hydroxyl derivative.

G'1 as a thiol, an amine or a hydroxyl derivative can be formalized as follow:

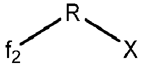

G'₁ as a maleimide can be formalized as follows:

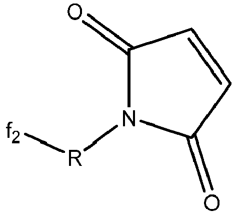

G'₁ as a vinylsulfone can be formalized as follows:

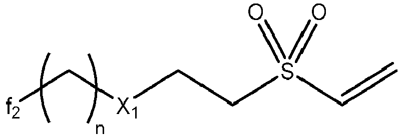

G'₁ as a strained cyclooctyne can be formalized as follows:

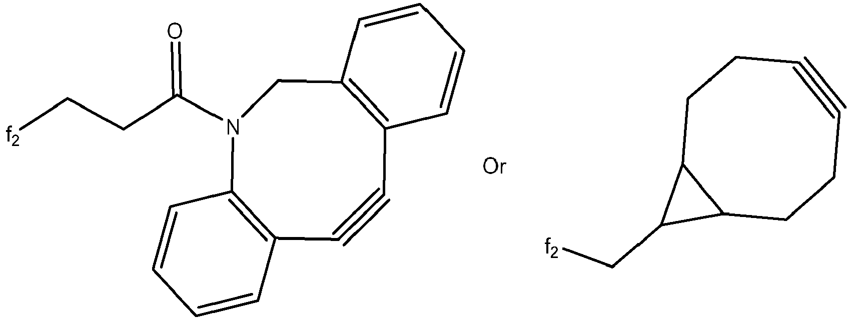

G'₁ as azide derivatives can be formalized with the following formula:

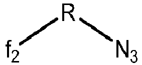

G'₁ as propargyl derivatives can be formalized with the following formula:

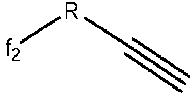

Wherein:
- n is an integer comprised from 0 to 7 (0 ≤ n₁ ≤ 7)
- R is a linear, branched, or cyclic alkyl derivative, or R is a PEG derivative
- X= -NH₂, or -OH, or -SH
- X₁ is an oxygen atom, or a sulphur atom, or a CH₂ group
- f₂ is defined as previously.

In an embodiment, if a=0, G'₁ is a propargyl derivative.

G'₁ as a propargyl derivative can be formalized as follow:

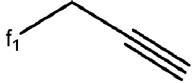

Wherein:
- f₁ is defined as previously

The invention also concerns a hydrogel comprising the cross-linked dextran polymer according to the invention.

In an embodiment the hydrogel is transparent.

By "transparent" is meant that in conditions disclosed in Example C21 of application PCT/EP2022/050466 for visual inspection an observer considered the sample transparent compared to the standard 2 (6 NTU) and/or the UV absorbance of the hydrogel as measured in Example C21 of application PCT/EP2022/050466 is lower than 0.06 (Absorbance Units).

In an embodiment the hydrogel is visually transparent and has a UV absorbance < 0.06 (Abs. Units).

In an embodiment the hydrogel according to the invention is characterized in that Tan δ is lower than 1.

In the present specification, Tan δ is the ratio of the storage modulus (also called elastic modulus) G' to the loss modulus G" (Tan δ = G'/G").

In an embodiment the hydrogel according to the invention is characterized in that Tan δ is less than or equal to 0.5.

In an embodiment the hydrogel according to the invention is characterized in that Tan δ is less than or equal to 0.1.

In an embodiment the hydrogel according to the invention is characterized in that Tan δ is less than or equal to 0.05.

In an embodiment the hydrogel according to the invention is characterized in that Tan δ is less than or equal to 0.01.

In an embodiment the hydrogel according to the invention is characterized in that after swelling in water the cross-linked dextran polymer concentration is comprised from 0.01 to 0.2 g/g.

In an embodiment the hydrogel according to the invention is characterized in that after swelling in water the cross-linked dextran polymer concentration is comprised from 0.03 to 0.1 g/g.

In an embodiment the hydrogel according to the invention is characterized in that after swelling in water the cross-linked dextran polymer concentration is comprised from 0.05 to 0.1 g/g.

In an embodiment, the hydrogel is translucid.

In another embodiment the hydrogel is transparent.

In an embodiment, the hydrogel has a Young modulus comprised between 1 to 200 kPa.

In an embodiment, the hydrogel has a Young modulus comprised between 5 to 200 kPa.

In an embodiment, the hydrogel has a Young modulus comprised between 20 to 200 kPa.

In an embodiment, the hydrogel has a Young modulus comprised between 30 to 200 kPa.

In an embodiment, the hydrogel has a Young modulus comprised between 50 to 200 kPa.

In an embodiment, the hydrogel has a Young modulus comprised between 30 to 180 kPa.

In an embodiment, the hydrogel has a Young modulus comprised between 50 to 150 kPa.

In an embodiment, the hydrogel has a Young modulus comprised between 5 to 100 kPa.

In an embodiment, the hydrogel has a Young modulus comprised between 10 to 90 kPa.

In an embodiment, the hydrogel has a Young modulus comprised between 10 to 75 kPa.

In an embodiment, the hydrogel has a G' comprised from 0.5 to 70 kPa.

In an embodiment the hydrogel has a compression deformation at break of more than or equal to 10 %.

In an embodiment the hydrogel has a compression deformation at break of more than or equal to 15 %.

In an embodiment the hydrogel has a compression deformation at break of more than or equal to 20 %.

In an embodiment the hydrogel has a compression deformation at break of more than or equal to 25 %.

In an embodiment the hydrogel has a compression deformation at break of more than or equal to 30 %.

In an embodiment the hydrogel has a compression deformation at break of more than or equal to 35 %.

In an embodiment the hydrogel has a compression deformation at break of more than or equal to 40 %.

In an embodiment the hydrogel has a compression deformation at break of more than or equal to 45 %.

In an embodiment the hydrogel has a compression deformation at break of more than or equal to 50 %.

In an embodiment the hydrogel has a compression deformation at break of more than or equal to 55 %.

In an embodiment the hydrogel has a compression deformation at break of more than or equal to 60 %.

In an embodiment the hydrogel has a traction deformation at break of more than or equal to 10 %.

In an embodiment the hydrogel has a traction deformation at break of more than or equal to 15 %.

In an embodiment the hydrogel has a traction deformation at break of more than or equal to 20 %.

In an embodiment the hydrogel has a traction deformation at break of more than or equal to 25 %.

In an embodiment the hydrogel has a traction deformation at break of more than or equal to 30 %.

In an embodiment the hydrogel has a traction deformation at break of more than or equal to 35 %.

In an embodiment the hydrogel has a traction deformation at break of more than or equal to 40 %.

In an embodiment the hydrogel has a swelling ratio of more than 0.7.

In an embodiment the hydrogel has a swelling ratio of more than 0.8.

In an embodiment the hydrogel has a swelling ratio of more than 0.9.

In an embodiment the hydrogel has a swelling ratio of more than 1.

In an embodiment the hydrogel has a swelling ratio of more than 1.1.

In an embodiment the hydrogel has a swelling ratio of more than or equal to 1.2.

In an embodiment the hydrogel has a swelling ratio of more than or equal to 1.3.

In an embodiment the hydrogel has a swelling ratio of more than or equal to 1.4.

In an embodiment the hydrogel has a swelling ratio of more than or equal to 1.5.

In an embodiment the hydrogel has a swelling ratio of more than or equal to 1.6.

In an embodiment the hydrogel has a swelling ratio of less than or equal to 5.

In an embodiment the hydrogel has a swelling ratio of less than or equal to 4.

In an embodiment the hydrogel has a swelling ratio of less than or equal to 3.

In an embodiment the hydrogel has a swelling ratio of less than or equal to 2.8.

In an embodiment the hydrogel has a swelling ratio of less than or equal to 2.5.

In an embodiment the hydrogel has a swelling ratio of less than or equal to 2.3.

In an embodiment the hydrogel has a water content of at least 80 wt%.

In an embodiment the hydrogel has a water content of at least 85 wt%.

In an embodiment the hydrogel has a water content of at least 90 wt%.

In an embodiment the hydrogel has a water content of at least 97 wt%.

In an embodiment the hydrogel has a water content of at least 96 wt%.

In an embodiment the hydrogel has a water content of at least 95 wt%.

In an embodiment the hydrogel has a water content of at least 94 wt%.

In an embodiment the hydrogel has a water content of at least 93 wt%.

In an embodiment the hydrogel has a water content of at most 99 wt%.

In an embodiment the hydrogel has a water content of at most 98 wt%.

In an embodiment the hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are proteins, hormones or peptide secreting cells.

In an embodiment the cells are chosen from:
- insulin secreting cells for diabetes treatment
- Factor VIII or Factor IX secreting cells for hemophilia treatment and
- β-glucocerebrosidase secreting cells for Gaucher disease.

In an embodiment the hydrogel according to the invention is characterized in that insulin secreting cells are chosen into the group of pancreatic cells.

In an embodiment the hydrogel according to the invention is characterized in that insulin secreting cells are Langherans islets.

In an embodiment the hydrogel according to the invention is characterized in that the biological cells are pseudoislets.

The invention also concerns the use of a cross-linked dextran copolymer according to the invention into the form of a hydrogel to prepare a cells composition.

In an embodiment the cells are chosen amongst one or multiple type of cells, either isolated or aggregated, which may secrete active principles.

In an embodiment, the cross-linked dextran polymer according to the invention is a dextran polymer bearing anionic groups of formula X wherein the at least divalent radical L(-)ᵢ is covalently bound to the dextran polymer backbone with i radicals, wherein i is 2, 4 or 8.

In an embodiment, the cross-linked dextran polymer according to the invention is a dextran polymer bearing anionic groups of formula X wherein the at least divalent radical L(-)ᵢ is covalently bound to the dextran polymer backbone with i radicals, wherein, i is 2.

In an embodiment, the cross-linked dextran polymer according to the invention is a dextran polymer bearing anionic groups of formula X wherein the at least divalent radical L(-)ᵢ is covalently bound to the dextran polymer backbone with i radicals, wherein, i is 4.

In an embodiment, the cross-linked dextran polymer according to the invention is a dextran polymer bearing anionic groups of formula X wherein the at least divalent radical L(-)ᵢ is covalently bound to the dextran polymer backbone with i radicals, wherein, i is 8.

The cross-linking step is a gelation step that leads to the formation of a hydrogel according to the invention.

The hydrogel formation kinetic is function of the temperature and could be modulated by the reactant's concentrations, pH and temperature.

In an embodiment the time to obtain a hydrogel according to the invention is comprised from 1 minute to 6 hours.

In an embodiment the cross-linking step is carried out for 1 hour.

In an embodiment the temperature of the cross-linking step is comprised from 4°C to room temperature (20-25°C) and could vary between the step of mixing and the step of gelation and moulding.

In an embodiment the mixing is performed at 4°C and the gelation is carried out at room temperature (20-25°C) for 1 hour.

In an embodiment the mixing is performed at room temperature (20-25°C).

In an embodiment the mixing is performed at 4°C or room temperature (20-25°C) and the gelation is carried out at room temperature (20-25°C) for 1 hour.

In an embodiment, the gelation is carried out at 37 °C.

In an embodiment, after cross-linking or gelation, the hydrogel is swelled in a buffer solution, the pH of the buffer solution is comprised from 5 to 8, preferably from 6 to 8 and more preferably from 6.8 to 7.5.

In an embodiment, the buffer solution is a PBS solution at pH 7.4.

In an embodiment, the buffer solution is a Tris solution at pH 7.4.

In an embodiment, the buffer solution is a Tris solution at pH 8.

In an embodiment the swelling allows the hydrogel mass being increased by 1, 2, 3 or 4 compared to its initial mass.

The invention also concerns a process to synthetize a cross-linked dextran polymer according to the invention, into the form of a hydrogel, comprising the steps of:
a) preparation of a sterile solution comprising a dextran bearing anionic groups of formula X and at least two precursors of -(A-f₂)ₐ-G₁-, -(A'-f₂)ₐ-G'₁-,
b) preparation of a sterile solution of a precursor of L(-)ᵢ
c) addition of the sterile solution obtained from step b) to the solution obtained from step a),
d) the addition being directly done in a mould or the solutions are introduced into a mould after being mixed,
e) cross-linking and gelation, for example at room temperature (20-25°C) or at 37°C,
f) unmoulding and swelling to obtain an hydrogel.

In an embodiment steps c) and d) are done simultaneously.

In an embodiment, the swelling is done into a PBS solution at pH 7,4.

Dextrans bearing anionic groups of formula X are prepared by grafting or substitution on the hydroxyl groups borne by the dextrans. In an embodiment the dextrans bearing anionic groups of formula X are prepared by grafting or substituting the carboxymethyl groups borne by the carboxymethyl dextrans.

In an embodiment of the process according to the invention an active pharmaceutical ingredient (API) is entrapped into the hydrogel.

The invention also concerns a therapeutic use of the hydrogel according to the invention as a therapeutic implant to administer the API to a mammal.

The invention also concerns a process to prepare a hydrogel comprising biological cells comprising the steps of:
a) preparation of a sterile solution comprising a dextran a dextran bearing anionic groups of formula X and at least two precursors of -(A-f₂)ₐ-G₁-, - (A'-f₂)ₐ-G'₁-,
b) preparation of a sterile solution of a precursor of L(-)ᵢ,
c) preparation of a suspension of biological cells,
d) mixing the biological cells suspension obtained from step c) and the solution obtained from the step b) or a),
e) addition of the sterile solution obtained from step a) or b) which is not used in step d) to the solution obtained from step d),
f) the addition of step e) being either done directly in a mould or the solutions are introduced into a mould after being mixed,
g) cross-linking and gelation reaction at room temperature (20-25°C),
h) unmoulding and swelling to obtain an hydrogel comprising biological cells.

In an embodiment, the swelling is done into a PBS solution at pH 7.4.

In an embodiment the hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are protein(s), hormone(s) or peptide(s) secreting cells.

In an embodiment the cells are chosen from
- insulin secreting cells for diabetes treatment
- Factor VIII or Factor IX secreting cells for hemophilia treatment and
- β-glucocerebrosidase secreting cells for Gaucher disease.

In an embodiment the hydrogel according to the invention is characterized in that insulin secreting cells are chosen into the group of pancreatic cells.

In an embodiment the hydrogel according to the invention is characterized in that insulin secreting cells are Langherans islets.

In an embodiment the hydrogel according to the invention is characterized in that the biological cells are pseudoislets.

The invention also concerns a therapeutic use of the hydrogel according to the invention for treating a disorder or disease in a mammal wherein the disorder or disease is due to lack or malfunction of endocrine function of pancreas organ.

The invention also concerns a hydrogel for use as a medicament.

The invention also concerns a hydrogel for use in the treatment of a disease such as diabetes.

The invention also concerns an implantable device comprising at least a hydrogel according to the invention and obtained according to the process of the invention.

The invention also concerns an implant consisting of the hydrogel according to the invention.

The invention also concerns an implant comprising the hydrogel according to the invention.

The invention also concerns an implant comprising the hydrogel according to the invention and cells or islets.

In an embodiment, at least 50 % of the surface the hydrogel is directly in contact with the medium in which it is implanted.

In an embodiment, at least 75 % of the surface the hydrogel is directly in contact with the medium in which it is implanted.

In an embodiment, at least 90 % of the surface the hydrogel is directly in contact with the medium in which it is implanted.

In an embodiment, at least 95 % of the surface the hydrogel is directly in contact with the medium in which it is implanted.

In an embodiment, 99 % of the surface the hydrogel is directly in contact with the medium in which it is implanted.

In an embodiment, at least 50 % of the surface the hydrogel is directly in contact with the exterior of the device or implant.

By « directly in contact with the exterior » means there is no separation between the hydrogel and the exterior, for example no wall made of a non-hydrogel material between the hydrogel and the exterior of the device or implant.

In an embodiment, at least 75 % of the surface the hydrogel is directly in contact with the exterior of the device or implant.

In an embodiment, at least 90 % of the surface the hydrogel is directly in contact with the exterior of the device or implant.

In an embodiment, at least 95 % of the surface the hydrogel is directly in contact with the exterior of the device or implant.

In an embodiment, at least 99 % of the surface the hydrogel is directly in contact with the exterior of the device or implant.

In an embodiment, 100 % of the surface the hydrogel is directly in contact with the exterior of the device or implant.

The cells or the API are entrapped into the maze of cross-linked dextran hydrogel.

In this specification the word "entrapped" is equivalent to "encapsulated" or "encapsulation".

The hydrogel matrix allows passage of small molecules e.g. nutrients and API, API being entrapped into the hydrogel or secreted by the entrapped cells.

Typically, API are hormone and peptide drugs chosen amongst PTH protein, insulin and coagulation factors.

In an embodiment, the mesh size of the matrix is immunoisolant and stops the T lymphocytes in order to preserve the cells.

In an embodiment, this mesh size is less than 1 µm.

In another embodiment it is less that 100 nanometers, preferably less than 10 nanometers, and more preferably around 5 nanometers.

In an embodiment the invention concerns an implant comprising a ring, a net, the hydrogel according to the invention and cells.

The ring and net structure allow the hydrogel to be easily manipulated, a good resistance to manipulation, including for implantation. This is also true even with a hydrogel having a larger mesh size (for example with a lower DS -(A-f₂)ₐ-G₁- and lower concentrations of reactive groups during crosslinking).

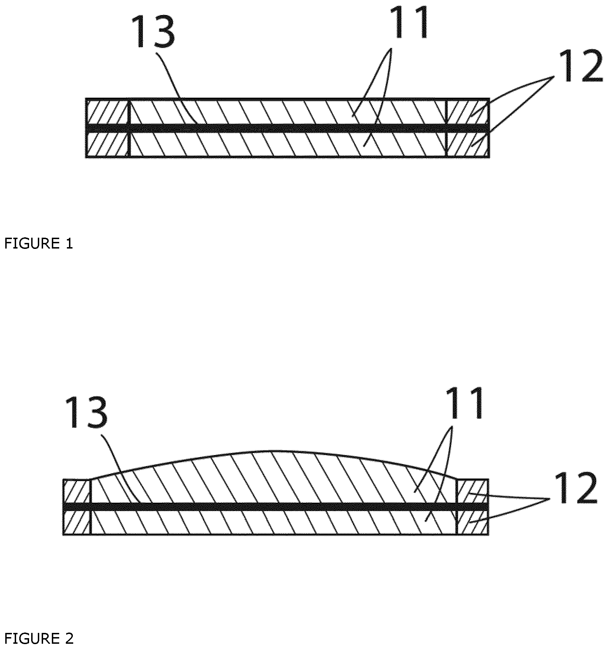

Figure 1 represents an implant (1) comprising a hydrogel (11) comprising cells or islets (not represented), a ring (12) and a net (13). Upper part of the ring, lower part of the ring and net can be glued together (not represented).
Figure 2 represents an implant (1) comprising a hydrogel (11) comprising cells or islets (not represented), a ring (12) and a net (13), where the hydrogel is concave.

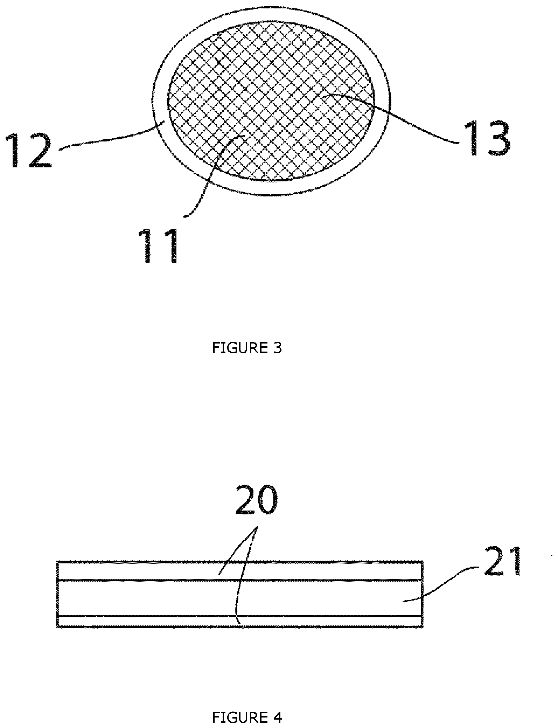

Figure 3 is a top view of an implant (1) comprising a hydrogel (11) comprising cells or islets (not represented), a ring (12) and a net (13). The upper part of 20 being optional.
Figure 4 represents an implant (1) comprising an hydrogel without cells (20) sandwiching an hydrogel comprising cells (21).

In an embodiment the ring has an internal diameter of 10 to 100 mm.

In an embodiment the ring has an internal diameter of 15 to 50 mm.

In an embodiment the ring has a diameter of 0.5 to 5 mm

In an embodiment the ring has a diameter of 0.5 to 10 mm.

In an embodiment the ring has a diameter of 0.5 to 5 mm.

In an embodiment the ring has a total (lower plus upper part and glue) thickness of 100 to 3000 µm.

In an embodiment the ring has a total (lower plus upper part and glue) thickness of 150 to 2000 µm.

In an embodiment the ring has a total thickness of 200 to 5000 µm.

In an embodiment the ring has a total thickness of 500 to 3000 µm.

In an embodiment the ring has a rectangular, square or round section.

In an embodiment the ring material is a bioinert material.

In an embodiment, the ring material is a biocompatible elastomer.

In an embodiment the ring material is chosen from the group consisting of silicone, in particular PDMS, polyurethanes, polyether, polyether polyester copolymers and polypropylene oxide.

In an embodiment the ring material is silicone.

In an embodiment the ring material is PDMS.

In an embodiment the net is non-biodegradable.

In an embodiment the net is biocompatible.

In an embodiment the net is non absorbable.

In an embodiment the net is a surgical mesh.

In an embodiment the filament material of the net material is chosen among the group consisting of Polypropylene, Polyethylene, polyester, in particular PET, PTFE, PVDF (polyvinylidene fluoride) and ePVDF (extended PVDF).

In an embodiment the filament material of the net is chosen among the group consisting of Polypropylene, polyester, in particular PET, PTFE and PVDF (polyvinylidene fluoride).

In an embodiment the filament material of the net is chosen among the group consisting of Polypropylene and polyester, in particular PET.

In an embodiment the filament material of the net is chosen among the group consisting of Polypropylene.

In an embodiment the filament material of the net is chosen among the group consisting of is polyester, in particular PET.

In an embodiment the net has a thickness ranging from 50 to 500 µm.

In an embodiment the m net has a thickness ranging from 100 to 300 µm.

In an embodiment, the filament diameter is ranging from 0.08 to 0.2 mm.

In an embodiment the pore size is ranging from 0.4 to 4 mm.

In an embodiment the pore size is ranging from 0.6 to 2 mm.

In an embodiment fabric of the net is chosen from the group consisting of knitted fabric, warp knitted fabric, woven fabric, non-woven fabric.

In an embodiment fabric of the net is chosen from the group consisting of warp knitted fabric, in particular multi-filament.

In an embodiment the net is treated in order to increase the hydrophilicity.

In an embodiment the net is treated with a base, in particular on polyester, more particularly on PET.

In an embodiment this treatment is functionalisation of the surface from reactive function, such as -OH, -COOH, and reactive molecules or polymer.

The grafted polymer could thus expose reactive functions for a further reaction with the hydrogel or a precursor of hydrogel, such as a thiol function.

In an embodiment this treatment is done by adsorption of synthetic polymers, such as poloxamers or polyvinyl pyrrolidone (PVP) or of natural polymers, such as collagen, or of surfactants after a chemical or physical treatment.

In an embodiment the net remains below the exterior end of the ring.

In an embodiment the net is not in contact with the exterior of the implant comprising a ring, a net and the hydrogel.

In an embodiment the glue is biocompatible.

In an embodiment the glue is a biocompatible silicone glue, such as Silbione MED ADH 4200 supplied by Elkem.

In an embodiment the glue remains below the exterior end of the ring.

For example, a warp knit Polyester surgical net fabric type PETKM3002 (1x0.9mm pore size) supplied by SurgicalNet^{™} was treated in NaOH 1M for 5 hours at 70°C and rinsed with deionized water and ethanol 96%. The treatment led to an increased hydrophilicity of the net fabric leading to an improved wetting with aqueous solutions.

For example, a ring net construct may be obtained following the process below:
- Biocompatible PDMS sheets supplied by Grace Biolabs or Interstate Speciality Product is cut in a form of a square incorporating a circular empty disc using a stainless steel punch,
- A part of the treated polyester surgical net described is introduced in between two square PDMS pieces: The two PDMS pieces and the surgical net are glued together with biocompatible silicone glue (Silbione MED ADH 4200 supplied by Elkem). The circular empty discs are aligned, and the surgical net is kept tense during gluing,
- then, the square construct is cut with a strainless steel punch to obtain the final object constituted by two PDMS rings sandwiching a surgical net and glued together, and
- The pieces are washed with a solution of poloxamer F127 at 1% and rinsed with water before steam sterilization.

In an embodiment, the implant can be obtained by the following process:
- Hydrogel compositions are incorporated in the Ring Mesh constructs. The concentrated polymer solutions are mixed with a pipette and a controlled volume of the mixture is introduced in a ring mesh construct adhering to a glass slide,
- Crosslinking leading to gelation is carried out. Then the Ring Mesh + Hydrogel composition is introduced in a Tris 150 mM /NaCl 30 mM / Cystein 10 mM solution at pH 8 or in PBS at pH 7.4,
- The hydrogel was rinsed with PBS solution without cysteine and further immersed in the PBS solution overnight at 37°C. The hydrogel piece was then stored in PBS solution at 4°C until being used.

Hydrogel/Ring Mesh implants can then be easily manipulated with tweezers and are foldable for the need of surgical implantation. Moreover, it is possible to fix the ring with sutures.

The hydrogel volume can be adjusted with the internal diameter and the thickness or the ring mesh construct. For the same ring mesh construct the hydrogel volume can be adjusted to control the convexity / concavity of the hydrogel above the ring level.

In an embodiment, the hydrogel comprises a first layer of the hydrogel wich does not comprise cells or islets and a second layer of the hydrogel which comprises cells or islets.

Such a structure may be obtained with a process as disclosed in this application, but with two steps of adding hydrogel precursors first step is adding hydrogel precursors without cells or islets as a first layer, and the second step is adding the hydrogel precursor with cells or islets while the gelation of the first step is not finished, in particular at a time corresponding to 5 to 25 % of the gelation time of the first hydrogel, as a second layer.

## Claims

1. Cross-linked dextran polymer Dx- bearing anionic groups wherein a least divalent radical L(-)ᵢ is covalently bound to the dextran polymer backbone with i radicals, chosen among the dextrans of formula I, 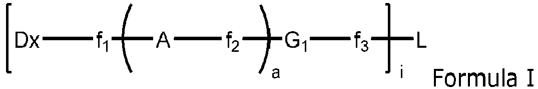 Wherein :
• a is an integer equal to 0 or 1.
• i is an integer comprised from 2 to 8, (2 ≤ i ≤ 8).
• L can be linked to the same [Dx-f₁-(A-f₂)ₐ-G₁-f₃] radicals, or to different ones.
• Dx- is a dextran moiety, which can be substituted by specific anionic groups in salified form, and optionally by alkyl carboxylate derivatives in salified form.
• f₁ is an ether function.
• The divalent radical -A- is a linear, -(CH₂)ₙ₁- with n₁ an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5)
• f₂ is an amide function.
• The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms. In a preferred embodiment, -G₁- is a succinimide derivative, or an alkyl sulfone derivative which can contain one heteroatom such as oxygen or sulphur, or a 1,4-triazole derivative.
• The integer i is the valence of the central-linker L, and the number of, identical or different, [Dx-f₁-(A-f₂)ₐ-G₁-f₃] radicals connected to L.
• f₃ is an amine function, or a thioether function, or an ether function, or an amide function, or a carbamate function, or a carbon-nitrogen covalent bond, or carbon-aromatic carbon covalent bond.
• The central-linker L is a poly(oxazoline) (POx) derivative, which can be linear or branched.

2. Cross-linked dextran polymer according to claim 1, wherein the anionic groups are chosen among alkyl carboxylate anions, sulphate anions, or sulfonate anions, or phosphate anions, or phosphonate anions.

3. Cross-linked dextran polymer according to claim 1, wherein the anionic groups are chosen among anionic groups of Formula X: 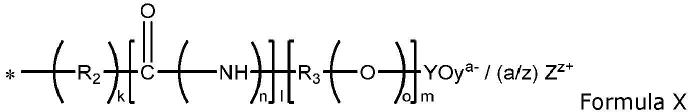 Wherein:
• ^{∗} represents the link to the O atoms of the dextran to form an ether function.
• y=2 or 3.
• When y=2, alkyl carboxylate derivatives, then:
∘ Y=C and a=1.
∘ k=1, l=0 and m=0.
∘ R2=Alkyl.
• When y=3, anionic group, then:
∘ Y=S and a=1, or Y=P and a=2.
∘ k=0 or 1.
∘ l=0 or 1.
∘ m=0 or 1.
∘ n=1 or 2, in particular 1.
∘ o=0 or 1.
∘ if l=1 then m=1.
∘ R₃=linear, branched, or cyclic alkyl which may contain one heteroatom such as nitrogen, or aromatic, or PEG.
∘ R2=Alkyl.
• And, Z is a counter ion, which can be an alkali metal and z=1, or which can be an alkaline earth metal and z=2.

4. Dextran polymers of formula VIII before the crosslinking reaction. 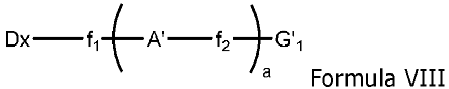 Wherein
• f₁, f₂, Dx are as defined above if a is not equal to 0,
• and
• if a is equal to 0, f₁ is an ether function and G'₁ is a propargylic derivative,
• if a is not equal to 0 -A' is A as defined above.

5. Process to synthetize a cross-linked dextran polymer according to the invention, into the form of a hydrogel, comprising the steps of:
a) preparation of a sterile solution comprising a dextran bearing anionic groups of formula X and at least two precursors of -(A-f₂)ₐ-G₁-, -(A'-f₂)ₐ-G'₁
b) preparation of a sterile solution of a precursor of L(-)ᵢ
c) addition of the sterile solution obtained from step b to the solution obtained from step a,
d) the addition being directly done in a mould or the solutions are introduced into a mould after being mixed,
e) cross-linking and gelation, for example at room temperature (20-25°C) or at 37°C,
f) unmoulding and swelling to obtain an hydrogel.

6. Process to prepare a hydrogel comprising biological cells comprising the steps of:
a) preparation of a sterile solution comprising a dextran a dextran bearing anionic groups of formula X and at least two precursors of -(A-f₂)ₐ-G₁-, -(A'-f₂)ₐ-G'₁-,
b) preparation of a sterile solution of a precursor of L(-)ᵢ,
c) preparation of a suspension of biological cells,
d) mixing the biological cells suspension obtained from step c and the solution obtained from the step b or a,
e) addition of the sterile solution obtained from step a or b which is not used in step d to the solution obtained from step d,
f) the addition of step e being either done directly in a mould or the solutions are introduced into a mould after being mixed,
g) cross-linking and gelation reaction at room temperature (20-25°C),
h) unmoulding and swelling to obtain an hydrogel comprising biological cells.

7. Hydrogel comprising the crosslinked dextran polymer according to any one of the preceding claims.

8. Hydrogel according to claim 9, **characterized in that** it further comprises biological cells.

9. Therapeutic use of the hydrogel according to any one of claims 9 to 10, for treating a disorder or disease in a mammal wherein the disorder or disease is due to lack or malfunction of endocrine function of pancreas organ

10. Implant comprising the hydrogel according to any one of claims 9 to 10
